Europäisches Patentamt

European Patent Office

Office européen des brevets

(11) Numéro de publication:

**0 193 444**

**A1**

## (12) DEMANDE DE BREVET EUROPEEN

(21) Numéro de dépôt: 86400267.0

(22) Date de dépôt: 07.02.86

(51) Int. Cl.4: **H05G 1/60** , G21K 1/10 , A61B 6/06

(30) Priorité: 12.02.85 FR 8501973

(43) Date de publication de la demande:
03.09.86 Bulletin 86/36

(84) Etats contractants désignés:
DE GB IT NL

(71) Demandeur: THOMSON-CGR
13, square Max-Hymans
F-75015 Paris(FR)

(72) Inventeur: Klausz, Rémy
THOMSON-CSF SPCI 19, avenue de Messine
F-75008 Paris(FR)

(74) Mandataire: Chaverneff, Vladimir et al
THOMSON-CSF SCPI 19, avenue de Messine
F-75008 Paris(FR)

(54) Installation de radiologie à compensation dans un trajet optique de l'image.

(57) Installation radiologie comportant un système de réglage de luminosité placé entre un récepteur tel qu'un amplificateur de luminance et une caméra de télévision.

Selon l'invention, les moyens de réglage comportent un diaphragme 24 à variation d'ouverture relativement faible et un atténuateur 26 dont la caractéristique d'atténuation dépend du rayon.

EP 0 193 444 A1

FIG_1

# INSTALLATION DE RADIOLOGIE A COMPENSATION DANS UN TRAJET OPTIQUE DE L'IMAGE

L'invention concerne une installation de radiologie à compensation dans un trajet optique de l'image, comportant un diaphragme de réglage de la "luminosité" globale de l'image ; elle concerne plus particulièrement un perfectionnement assurant en toute circonstance une meilleure qualité d'image et permettant de réduire en outre le temps de réponse d'un tel diaphragme.

Une installation de radiologie classique comporte une source de rayons X et un récepteur fournissant une image optique, comme par exemple un amplificateur de luminance. Le patient est placé entre la source et le récepteur. L'amplificateur de luminance comporte un écran de sortie donnant de l'image radiologique une image lumineuse reprise par une caméra de télévision, pour son exploitation et sa visualisation sur un tube cathodique.

Un système de transmission optique est intercalé entre cet écran de visualisation et l'objectif de la caméra. Il comprend classiquement une première lentille ou objectif positionné de façon qu'un plan focal de celui-ci soit confondu avec le plan de l'écran de sortie pour en donner une image "à l'infini" laquelle est reprise par une seconde lentille ou objectif reformant une image réelle sur la cible du tube analyseur de la caméra. Un diaphragme à iris, commandé par un moteur électrique permet de régler la quantité de lumière transmise à la caméra pour s'adapter, d'une part, à la luminosité moyenne de l'image formée sur l'écran de visualisation et, d'autre part, pour s'adapter aux différents modes d'exploitation de l'image : radioscopie, radiographie numérique, etc ... L'utilisation de ce diaphragme pose deux problèmes. D'une part, le temps nécessaire pour passer d'une ouverture de diaphragme à une autre est parfois trop long pour des prises de vues à cadence élevée, notamment en angiographie. Il faut par exemple compter 0,5 secondes pour passer d'une position correspondant à la radioscopie à une position correspondant à la radiographie numérique. D'autre part, un fonctionnement à "dose" élevée, engendrant sur l'écran de sortie de l'amplificateur de luminance une image très lumineuse, amène à réduire considérablement l'ouverture du diaphragme. Il en résulte une dégradation très sensible de la qualité de l'image. En effet, les meilleures images sont obtenues pour des ouvertures de diaphragme comprises entre la moitié et le tiers de l'ouverture maximum.

L'invention permet de résoudre ce double problème.

Dans cet esprit, l'invention concerne essentiellement une installation de radiologie du type comportant une source de rayons X et un récepteur tel que par exemple un amplificateur de luminance, une caméra de télévision et des moyens de transmission optique intercalés entre une sortie de visualisation dudit récepteur et ladite caméra, ces moyens de transmission optiques comportant un diaphragme commandé placé dans une zone "à l'infini" du trajet optique, caractérisée en ce qu'un atténuateur optique dont la caractéristique d'atténuation diminue radialement à partir de son centre est disposé en regard dudit diaphragme dans ladite "zone à l'infini" dudit trajet optique, de façon que ledit centre soit situé sur l'axe dudit diaphragme.

Dans la définition ci-dessus, la zone "à l'infini" désigne la portion de trajet optique située entre les deux lentilles ou objectifs des moyens de transmission optique précités. Avec une telle structure, les variations de diamètre de l'ouverture de diaphragme peuvent être le plus souvent maintenues dans la plage préférentielle définie ci-dessus. En outre, le temps de passage d'une ouverture de diaphragme à une autre est notablement réduit.

L'invention sera mieux comprise et d'autres avantages de celle-ci apparaîtront mieux à la lumière de la description qui va suivre, donnée uniquement à titre d'exemple et faite en référence aux dessins annexés dans lesquels :

-la figure 1 est une vue schématique de l'ensemble d'une installation conforme à l'invention ;

-la figure 2 représente un premier mode de réalisation de l'atténuateur utilisé dans l'installation de la figure 1 ;

-la figure 3 représente un second mode de réalisation possible de cet atténuateur.

En se reportant aux dessins, on a représenté une installation de radiologie comportant une source de rayons X 11 émettant un faisceau de rayons X 12 en direction d'un récepteur 13 constitué ici par un amplificateur de luminance. L'écran de sortie 14, ou écran secondaire, de cet amplificateur de luminance est analysé par une caméra de télévision 15 dont le signal vidéo est transmis d'une part à un récepteur de télévision 16 permettant une radioscopie directe et d'autre part à un système d'angiographie numérique 17 agencé pour appliquer un certain nombre de traitements numériques à une séquence d'images enregistrées sous forme numérique (notamment des soustractions logarithmiques d'images). Le résultat des traitements est visualisé sur un autre récepteur de télévision 18 associé au système d'angiographie numérique 17. Des moyens de transmission optique 20 sont intercalés entre l'écran 14 et la caméra 15. Ces moyens de transmission comprennent notamment deux lentilles ou objectifs 21, 22 ayant un axe optique 23 commun. Un diaphragme à iris 24 centré sur ce même axe est placé dans le trajet optique dit "à l'infini" entre les deux lentilles ou objectifs. L'ouverture du diaphragme est commandé par un moteur électrique 25. Selon l'invention, les moyens de transmission optique 20 comportent en outre un atténuateur optique 26 disposé en regard du diaphragme 24 dans ladite "zone à l'infini" du trajet optique, c'est-à-dire comme le diaphragme, entre les deux lentilles ou objectifs 21, 22. La caractéristique d'atténuation de cet atténuateur est la même sur tout cercle de centre 0 et ce centre est situé sur l'axe optique 23, confondu avec l'axe du diaphragme. Par ailleurs, cette caractéristique d'atténuation diminue radialement à partir du centre 0.

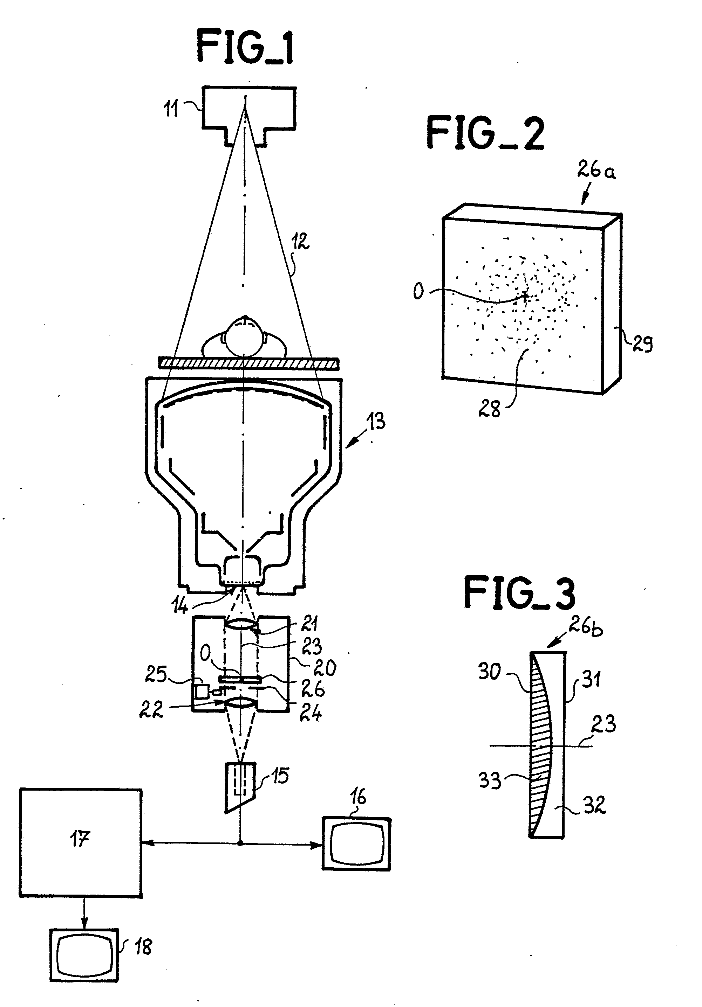

Selon un premier mode de réalisation possible, représenté à la figure 2, l'atténuateur 26a peut être réalisé par un dépôt 28 d'opacité uniforme sur tout cercle de centre O mais diminuant radialement, ce dépôt étant effectué sur une simple lame transparente 29, formant support. Le dépôt pourra par exemple être un dépôt métallique d'épaisseur décroissant radialement à partir du centre 0. Une autre façon de réaliser l'atténuateur est illustrée à la figure 3. Comme représenté, l'atténuateur 26b consiste en une lame à faces parallèles 30, 31 comportant deux parties plano-sphériques 32, 33, l'une concave l'autre convexe, de même rayon de courbure et imbriquées l'une dans l'autre. Les deux parties 32 et 33 présentent le même indice de réfraction. La partie du type plan-concave 32 est réalisée en matériau transparent tandis que la partie du type plan-convexe 33 présente une atténuation linéaire prédéterminée. Ainsi l'atténuation d'un rayon lumineux traversant cet agencement parallèlement à l'axe 23 dépendra de l'épaisseur de la partie plan-convexe traversée par lui, c'est-à-dire de la distance séparant ce rayon de l'axe 23.

On peut en outre envisager de réaliser l'atténuateur sous la forme d'un ou plusieurs disques de centre O, ayant une atténuation uniforme, éventuellement totale et dont le ou les diamètres sont compris entre le diamètre d'ouverture maximum et le diamètre d'ouverture minimum du diaphragme. La caractéristique d'atténuation diminue alors radialement par palier(s) à partir du centre O.

Comme indiqué précédemment, l'ouverture du diaphragme peut le plus souvent être maintenue dans la plage de variation optimale du point de vue de la qualité optique de l'image transmise. En effet, la diaphragme règle la quantité de lumière transmise par la variation de diamètre de son ouverture. Or l'atténuateur à absorption variable radialement fait lui-même varier la transmission moyenne de lumière en fonction de l'ouverture de diaphragme. L'effet global obtenu est donc une variation totale de transmission de lumière plus importante pour une même variation de diamètre du diaphragme. En conséquence, le temps nécessaire pour passer d'une position d'ouverture quelconque à une autre par commande du moteur électrique 25, est notablement réduit.

## Revendications

1. Installation de radiologie du type comportant une source de rayons X (11) et un récepteur (13) tel que par exemple un amplificateur de luminance, une caméra de télévision (15) et des moyens de transmission optique (20) intercalés entre une sortie de visualisation (14) dudit récepteur et de ladite caméra, ces moyens de transmission optique comportant un diaphragme commandé (24), placé dans une zone "à l'infini" dudit trajet optique, caractérisée en ce qu'un atténuateur optique (26) dont la caractéristique d'atténuation diminue radialement à partir de son centre - (O) est disposé en regard dudit diaphragme dans ladite "zone à l'infini" dudit trajet optique de façon que ledit centre soit situé sur l'axe dudit diaphragme.

2. Installation de radiologie selon la revendication 1, caractérisé en ce que ledit atténuateur consiste en un dépôt (28) par exemple métallique, d'opacité variable radialement, sur une lame transparente (29) formant support.

3. Installation de radiologie selon la revendication 1, caractérisée en ce que ledit atténuateur consiste en une lame à faces parallèles comportant deux parties plano-sphériques (32, 33) de même rayon de courbure et imbriquées l'une dans l'autre, l'une (32) des parties étant du type plan-concave et réalisée en matériau transparent, et l'autre (33) étant du type plan-convexe, de même coefficient de réfraction et présentant une atténuation linéaire prédéterminée.

4. Installation de radiologie selon la revendication 1, caractérisée en ce que ledit atténuateur comporte au moins un disque d'atténuation uniforme, de diamètre compris entre le diamètre d'ouverture maximum et le diamètre d'ouverture minimum dudit diaphragme.

0 193 444

FIG_1

FIG_2

FIG_3

Office européen
des brevets

**RAPPORT DE RECHERCHE EUROPEENNE**

Numéro de la demande

EP    86 40 0267

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | DE-A-2 846 295  (SIEMENS AG)<br>* Page 1, lignes 3-9, 28-33; figure 1 *<br><br>--- | 1 | H 05 G    1/60<br>G 21 K    1/10<br>A 61 B    6/06 |
| A | FR-A-2 179 039  (SIEMENS AG)<br>* Page  4,  ligne  39  - page 6, ligne 32; figure *<br><br>--- | 1 | |
| A | EP-A-0 087 843  (N.V. PHILIPS' GLOEILAMPENFABRIEKEN)<br>* Page  8,  lignes 1-16; page 8, ligne  27 - page 9, ligne 6; figures 1,2 *<br><br>--- | 1 | |
| A | EP-A-0 126 434  (KABUSHIKI KAISHA TOSHIBA)<br>* Page 1, ligne 1 - page 2, ligne 34; figure 3 *<br><br>--- | 1 | DOMAINES TECHNIQUES RECHERCHES (Int. Cl.4) |
| A,P | EP-A-0 158 382  (N.V. OPTISCHE INDUSTRIE "DE OUDE DELFT")<br>* Page  7,  ligne  17  - page 9, ligne  11;  page 23, lignes 1-25; figures 1,2 *<br><br>----- | 1 | A 61 B    6/00<br>G 21 K    1/00<br>H 05 G    1/00 |

Le présent rapport de recherche a été établi pour toutes les revendications

| Lieu de la recherche<br>LA HAYE | Date d'achèvement de la recherche<br>15-05-1986 | Examinateur<br>HORAK G.I. |
|---|---|---|